# EUROPEAN PATENT SPECIFICATION

(11) **EP 0 413 731 B1**
(45) Date of publication and mention of the grant of the patent: **16.03.1994**
(21) Application number: 89905335.9
(22) Date of filing: 14.04.1989
(51) Int. Cl.: A61K 37/26, A61K 37/02, A61K 37/66

(54) **CHIMERIC PEPTIDES FOR NEUROPEPTIDE DELIVERY THROUGH THE BLOOD-BRAIN BARRIER**
SCHIMÄRISCHE PEPTIDE FÜR DIE LIEFERUNG VON NEUROPEPTIDEN DURCH DIE BLUT-HIRN-BARRIERE
PEPTIDES CHIMERIQUES D'ADMINISTRATION DE NEUROPEPTIDES A TRAVERS LA BARRIERE VASCULAIRE DU CERVEAU

(30) Priority: 25.04.1988 US 185702
(43) Date of publication of application: 27.02.1991
(73) Proprietor: THE REGENTS OF THE UNIVERSITY OF CALIFORNIA, Berkeley, California 94720-1620 (US)
(72) Inventor: PARDRIDGE, William, M., Pacific Palisades, CA 90272 (US); SCHIMMEL, Paul, R., Lexington, MA 02173 (US)
(74) Representative: Silverman, Warren
(86) International application number: PCT/US89/01589
(87) International publication number: WO 89/10134

(56) References cited:
- WO-A-88/00834
- Chemical Abstracts, vol. 107, no. 12, 21 Sept. 1987 (Columbus, Ohio, US), W.M. Pardridge et al., p. 379, abstract no. 102538b

## Description

The present invention relates generally to the introduction of neuropharmaceutical agents into the brain by transcytosis across the blood-brain barrier. More particularly, the present invention relates to chimeric peptides which are capable of transporting neuropharmaceutical agents into the brain by receptor-mediated transcytosis across the blood-brain barrier.

The vertebrate brain has a unique capillary system which is unlike that in any other organ in the body. The unique capillary system has morphologic characteristics which make up the blood-brain barrier (BBB). The blood-brain barrier acts as a systemwide cellular membrane which separates the brain interstitial space from the blood.

The unique morphologic characteristics of the brain capillaries which make up the BBB are: (a) epithelial-like high resistance tight junctions which literally cement all endothelia of brain capillaries together, and (b) scanty pinocytosis or transendothelial channels, which are abundant in endothelia of peripheral organs. Due to the unique characteristics of the blood-brain barrier, hydrophilic drugs end peptides that readily gain access to other tissues in the body are barred from entry into the brain or their rates of entry are very low.

Various strategies have been developed for introducing those drugs into the brain which otherwise would not cross the blood-brain barrier. The most widely used strategies involve invasive procedures where the drug is delivered directly into the brain. The most common procedure is the implantation of a catheter into the ventricular system to bypass the blood-brain barrier and deliver the drug directly to the brain. These procedures have been used in the treatment of brain diseases which have a predilection for the meninges, e.g., leukemic involvement of the brain.

Although invasive procedures for the direct delivery of drugs to the brain ventricles have experienced some success, they have not been entirely successful because they only distribute the drug to superficial areas of the brain tissues, and not to the structures deep within the brain. Further, the invasive procedures are potentially harmful to the patient.

Other approaches to circumventing the blood-brain barrier utilize pharmacologic-based procedures involving drug latentiation or the conversion of hydrophilic drugs into lipid-soluble drugs. The majority of the latentiation approaches involve blocking the hydroxyl, carboxyl and primary amine groups on the drug to make it more lipid-soluble and therefore more easily transported across the blood-brain barrier. Although the pharmacologic approaches have been used with some success, they may not be entirely satisfactory for delivery of peptides through the BBB based on the inventor's experience with cyclosporin transport through the BBB. Cyclosporin is a highly latentiated (lipid-soluble) peptide that crosses the BBB relatively slowly

Another approach to circumventing the blood-brain barrier involves the intra-arterial infusion of hypertonic substances which transiently open the blood-brain barrier to allow passage of hydrophilic drugs. However, hypertonic substances are potentially toxic and may damage the blood-brain barrier.

There presently is a need to provide improved substances and methods for delivering hydrophilic drugs and peptides across the blood-brain barrier and into the brain. It is desirable that such improved substances and methods provide for uniform introduction of the hydrophilic peptide or drug throughout the brain and present as little risk to the patient as possible.

In accordance with the present invention, new procedures and substances are disclosed which provide uniform distribution of neuropeptides and other drugs throughout the brain while reducing the problems inherent in prior invasive and pharmacologic drug introduction procedures.

The present invention is based on the surprising discovery that hydrophilic peptides may be physiologically transported across the blood-brain barrier by coupling or conjugating the drug to a transportable peptide which is capable of crossing the blood-brain barrier by receptor-mediated transcytosis. This discovery is particularly surprising in view of the traditional notion that the blood-brain barrier is a passive barrier which is impenetrable by hydrophilic drugs or peptides.

The invention involves novel chimeric peptides which are adapted to deliver a neuropharmaceutical agent into the brain by transcytosis across the blood-brain barrier. The chimeric peptides include a transportable peptide that is capable of crossing the blood-brain barrier at relatively high rate by receptor-mediated transcytosis. The transportable peptide is conjugated with a neuropharmaceutical agent to form the chimeric peptide. The neuropharmaceutical agent is generally a hydrophilic peptide that does not by itself significantly cross the BBB. The conjugation of transportable peptides with neuropharmaceutical agents was surprisingly found to produce chimeric peptides which were capable of being transported across the blood-brain barrier.

Histones are a group of naturally occurring proteins which have been found to be well suited for use as a transportable peptide in accordance with the present invention. Since histones are naturally occurring substances, they do not require organic synthesis and the possibility of an immune response associated with synthetically derived materials is greatly reduced.

The chimeric peptides are believed to be transported across the blood-brain barrier by the physiologic process of transcytosis via receptors in the blood-brain barrier. This insures that the chimeric peptide is distributed uniformly to all parts of the brain. In addition, the introduction of the chimeric peptide into the brain by a physiologic pathway reduces the harmful side effects and risks inherent in the traditional invasive and pharmacological approaches.

The present invention also includes methods for administering the chimeric peptides subcutaneously or intranasally and the chimeric peptide containing compositions utilized in such methods of treatment.

The above-discussed and many other features and attendant advantages of the present invention will become apparent as the invention becomes better understood by reference to the following detailed description when considered in conjunction with the accompanying drawing.

Fig. 1 is a chart showing the results of the tests described in Example No. 2.

Fig. 2 is a chart depicting the results of tests in accordance with example 10 showing the uptake of histone by brain capillaries.

Fig 5. 3a and 3b are charts depicting the results of histone uptake tests in accordance with example 10 which show the temperature and time dependence of the transport mechanism.

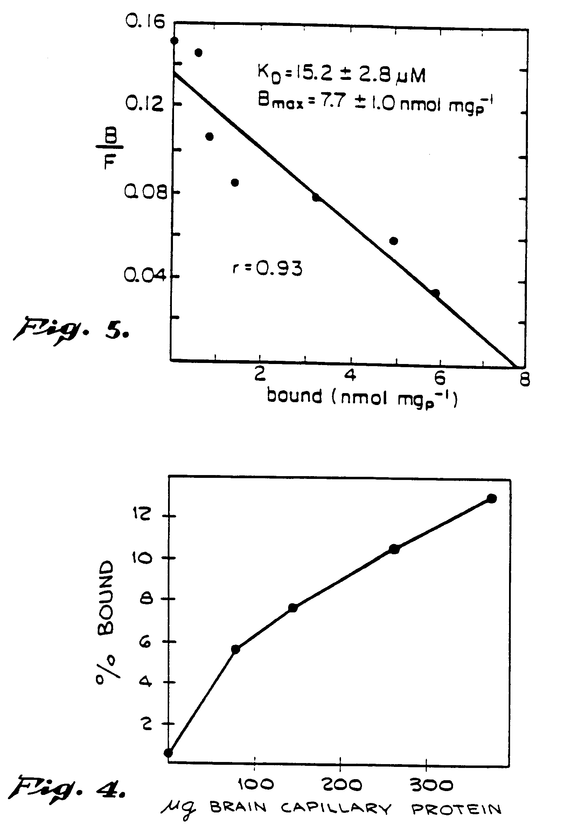

Fig. 4 is a chart showing the linearity of histone with respect to the amount of capillary protein.

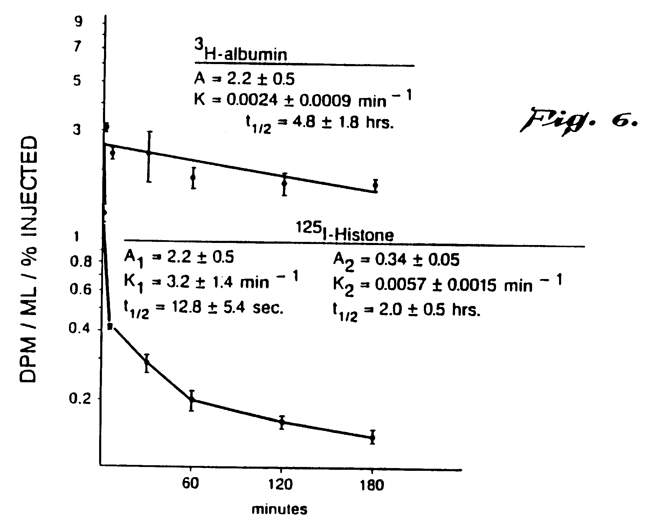

Fig. 5 depicts charts wherein the binding (% bound/mgₚ) of [¹²⁵I]-histone is plotted versus the concentration of unlabeled histone in the incubation vessel and wherein bound (B)/free (F) is plotted versus the [¹²⁵I]-histone bound to the bovine brain capillaries (nmol/mgₚ).

Fig. 6 is a chart showing the serum [³H]-albumin and [¹²⁵I]-histone radioactivity, A(t), (DPM/ml/percent injected) plotted versus time after a single intravenous injection of both isotopes.

The chimeric peptides in accordance with the present invention are useful in delivering a wide variety of neuropharmaceutical agents to the brain. The invention is particularly well suited for delivering neuropharmaceutical agents which are hydrophilic peptides. These hydrophilic peptides are generally not transported across the blood-brain barrier to any significant degree.

Exemplary hydrophilic peptide neuropharmaceutical agents are: thyrotropin releasing hormone (TRH) - used to treat spinal cord injury and Lou Gehrig's disease; vasopressin - used to treat amnesia; alpha interferon-used to treat multiple sclerosis; somatostatin - used to treat Alzheimer's disease; endorphin - used to treat pain; L-methionyl (sulfone)-L-glutamyl-L-histidyl-L-phenylalanyl-D-lysyl-L-phenylalanine (an analogue of adrenocorticotrophic hormone (ACTH)-4-9) - used to treat epilipsy; and muramyl dipeptide - used to treat insomnia. All of these neuropharmaceutical peptides are available commercially or they may be isolated from natural sources by well-known techniques.

The following description will be limited to chimeric peptides in which the neuropharmaceutical agents are hydrophilic peptides (neuropeptides) with it being understood that the invention has application to any neuropharmaceutical agent which by itself is transported at a low or non-existent rate across the blood-brain barrier. The invention also has application where it is desired to increase the rate at which the neuropharmaceutical agent is transported across the blood-brain barrier.

The chimeric peptide includes the hydrophilic peptide drug conjugated to a transportable peptide which is capable of crossing the blood-brain barrier by transcytosis at a much higher rate than the hydrophilic neuropeptides. Suitable transportable peptides include: histone, insulin, transferrin, insulin-like growth factor I (IGF-I), insulin-like growth factor II (IGF-II), basic albumin and prolactin.

Transferrin is an 80K glycoprotein that is the principal iron transport protein in the circulation. Transferrin is also a protein that is enriched in the cerebrospinal fluid (CSF). Transferrin is widely available and may be purchased or isolated from blood or CSF by well-known procedures.

Insulin, IGF-I and IGF-II are also commonly available. Insulin is available an a wide scale commercially and may also be recovered from natural sources by well-known techniques. IGF-I and IGF-II are available from commercial outlets such as Amgen or Peninsula Labs or they may be isolated from natural sources according to the procedure of Rosenfeld et al. (J. Clin Endocrinol. Metab. 55, 434, 1982).

Basic albumin or cationized albumin has a isoelectric point (pI) of 8.5 as compared to a pI of 3.9 for natural albumin. Cationized albumin, unlike natural albumin, enters the brain rapidly across the blood-brain barrier. Cationized albumin (pI = 8.5) is prepared preferably by covalent coupling of hexamethylene-diamine (HMD) to bovine serum albumin (pI = 3.5) according to Bergmann, et al., "Cationized Serum Albumin Enhances Response of Cultured Fetal Rat Long Bones To Parathyroid Hormone", Endocrinology, 116:1729-1733 (1985). An exemplary synthesis is as follows: 10 ml of a 10% solution of albumin in water is slowly added to 60 ml of 2.0 M HMD and the pH of the solution is adjusted to 6-7 with 1N HCl. After 30 minutes, 1 g of N-ethyl-N'-3-(dimethylaminopropyl) carbodiimide hydrochloride (EDAC) is added to activate the carboxyl groups of the albumin, followed by the addition of another 1 g EDAC 1 hour later. The pH is constantly adjusted to 6-7 with 0.2N HCl. The solution is allowed to stand overnight with constant stirring. The next day the solution is dialyzed extensively against distilled water. This solution is then purified by chromatofocusing using the Pharmacia polybuffer exchanger 94 resin and the polybuffer 96 elution buffer.

Histone is especially well suited for use as a transportable peptide because it is a naturally occurring protein that does not require organic synthesis such as the above procedure for preparing basic albumin. Further, the absence of antibody response to the naturally occurring histone makes it suitable in many situations where immune responses to synthesized materials, such as cationized albumin, would potentially limit its utility. Histones are a group of lysine-rich, highly cationic proteins that are subdivided into five classes (H1, H2, H3, H4, and H5). There are multiple subtypes in each of the five classes. These proteins are found in the nucleus of all cells and are tightly bound to the phosphate groups of chromatin. The histone molecules play a vital role in chromatin organization. The histones are routinely isolated from the acid-soluble fraction of nuclei isolated from calf thymus, chicken erythrocytes, or other starting materials. The different subtypes are separated by a number of well known techniques, such as isoelectric focusing or ion-exchange chromatography.

One characteristic of the histone molecules which is important for linkage chemistry is that the histones, with the exception of H3, lack a cysteine sulfhydryl group. Histones are known to undergo an extensive number of chemical modifications within the normal cell that include N-methylation, O-methylation, acetylation, phosphorylation, adenosine diphosphate (ADP) ribosylation, ubiguitination, and enzymatic hydrolysis of specific peptide bonds.

Histones are available from a wide variety of commercial sources or they may be isolated according to known procedures set forth in the following references:
Wu, R.S., Panusz, H.T., Hatch, C.L., and Bonner, W.M. (1986): Histones and their modifications. CRC Crit. Rev. Biochem. 20: 201-263; and
Coles, L.S., Robins, A.J., Madley, L.K., and Wells, J.R.E. (1987): Characterization of the chicken histone H1 gene complement. J. Biol. Chem. 262: 9656-9663.

Histones isolated from any of the conventional sources may be used and the particular class or subtype is also not particularly critical. It is preferred that histones isolated from human sources be used for preparing chimeric peptides for use in treating humans.

Another suitable transportable peptide is prolactin. Prolactin is a hormone which is secreted by the anterior pituitary. Prolactin is widely available commercially or it can be isolated from pituitary glands by well-known procedures.

The chimeric peptides are made by conjugating a transportable peptide with the neuropharmaceutical peptide. The conjugation may be carried out using bifunctional reagents which are capable of reacting with each of the peptides and forming a bridge between the two. The preferred method of conjugation involves peptide thiolation wherein the two peptides are treated with a reagent such as N-Succinimidyl 3-(2-pyridyldithio) propionate (SPDP) to form a disulfide bridge between the two peptides to form the chimeric peptide. Other known conjugation agents may be used, so long as they provide linkage of the two peptides (i.e. the hydrophilic peptide drug and the transportable peptide) together without denaturing them. Preferably, the linkage can be easily broken once the chimeric peptide has entered the brain. Suitable examples of conjugation reagents include: glutaraldehyde and cystamine and EDAC. Conjugation of peptides using glutaraldehyde is described in Poznansky et al., Insulin: Carrier potential for enzyme and drug therapy. Science 223:1304-1306, 1984. Conjugation of peptides using cystamine and EDAC is described in Ito et al., Transmembrane delivery of polypeptide hormones bypassing the intrinsic cell surface receptors: a conjugate of insulin with a2-macroglobulin (a2M) recognizing both insulin and a2M receptors and its biological activity in relation to endocytic pathways. Mol Cell Endocrinol 36:165, 1984.

Examples of preferred chimeric peptides include those having the general structure 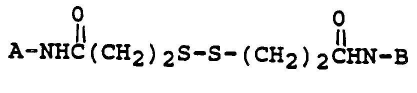
where A is somatostatin, thyrotropin releasing hormone (TRH), vasopressin, alpha interferon, endorphin, muramyl dipeptide or ACTH 4-9 analogue; and B is histone, insulin, IGF-I, IGF-II, transferrin, cationized (basic) albumin or prolactin.

Other examples of preferred chimeric peptides include those listed above wherein the disulfide conjugating bridge between A and B is replaced with bridges having the following structures:

A-NH(CH₂)₂S-S-B (cleavable linkage)

which are formed when cystamine and EDAC are employed as the bridge reagents;

A-NH=CH(CH₂)₃CH=NH-B (non-cleavable linkage)

which are formed when glutaraldehyde is employed as bridge reagent.

The chimeric peptides can be introduced into the body by any conventional procedure including parenteral injection or intranasal inhalation. Preferably, the chimeric peptides are combined with a compatible pharmaceutical carrier and injected parenterally or if desired combined with a suitable carrier and administered intranasally in accordance with the well-known conventional procedures used for intranasal administration of insulin. Suitable carrier solutions include those commonly used in injectable or nasal-inhaled hormone preparations such as sterile saline at a pH of around 5 which includes common bacteriostatic agents. The concentration of a chimeric peptide in the carrier will vary depending upon the specific transportable peptide and the specific neuropharmaceutical peptide. Preferably, levels of the chimeric peptide in the carrier should be between about 0.001 weight percent to 0.01 weight percent. As a general rule, the dosage levels and percent of chimeric peptides present in the injection or intranasal solution should correspond to the accepted and established dosages for the particular neuropharmaceutical peptide as well as the transportable peptide.

Examples of practice are as follows:

### Example 1 - Synthesis of Somatostatin-Insulin Chimera

Somatostatin, a peptide deficient in the brain of Alzheimer's disease, is a peptide which is not transported through the blood-brain barrier. Conversely, insulin is a peptide that is transported through the blood-brain barrier. The transportability of insulin through the blood-brain barrier is set forth in my article entitled "Receptor-Mediated Peptide Transport Through The Blood-Brain Barrier" (Endocrine Reviews, Vol. 7, No. 3, August 1986), the contents of which is hereby incorporated by reference.

Somatostatin and insulin were conjugated by peptide thiolation using a reversible peptide-peptide conjugation method as described by Carlsson, et al. in "Protein Thiolation and Reversible Protein-Protein Conjugation" (Biochem. J. (1978) 173, 723-737). A heterobifunctional reagent, N-succinimidyl 3-(2-pyridyldithio)propionate (SPDP), was used to couple a lysine or free N-terminus on insulin to a free lysine or amino terminus on somatostatin. Approximately 0.3 mg of insulin and 26 uCi of ¹²⁵I-insulin in 2 ml of phosphate buffered saline was prepared. To half of this was added 4 lambdas of 20 mM fresh SPDP and this was incubated at room temperature for 45 minutes.

Separately, 180 uCi of tritiated somatostatin in 180 uL of 0.01 N HCl was solubilized and added to 180 uL of 0.2 M phosphate buffered saline. To half of this, 4 uL of 20 mM SPDP was added and this was incubated for 45 minutes, followed by acidification with 20 uL of 0.75 M sodium acetate (pH = 4.5) followed by reduction with 20 uL of 0.25 M dithiothreitol. This was incubated at room temperature for 30 minutes followed by brief dialysis to remove unreacted small molecules. The conjugated insulin and conjugated somatostatin were then incubated overnight at room temperature followed by dialysis and counting for tritium and ¹²⁵I radioactivity. This resulted in a total of 53 uCi of ³H-somatostatin coupled to 5.3 uCi of ¹²⁵I-insulin in 2 ml of phosphate buffered saline.

The structure of the somatostatin-insulin chimera is shown below. 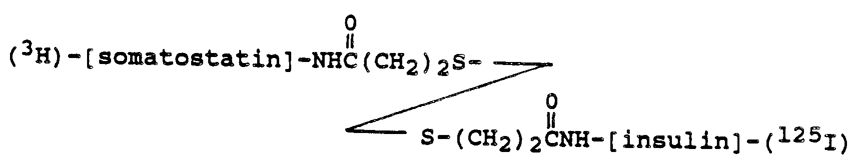
Somatostatin has the following amino acid sequence: Ala-Gly-Cys-Lys-Asn-Phe-Phe-Trp-Lys-Thr-Phe-Thr-Ser-Cys. Insulin is a double chain protein hormone whose structure is well known.

### Example 2 - Radioreceptor Assay Using Isolated Bovine Brain Microvessels and ³H-Somatostatin-¹²⁵I-Insulin Chimera

Somatostatin was obtained from Peninsula Laboratories and tritiated by reductive methylation using ³H-sodium borohydride. Insulin was obtained from Sigma Chemical Company and was iodinated by oxidative iodination using chloramine T and ¹²⁵I-iodine. The two compounds were coupled together using SPDP as described in Example 1. Bovine brain microvessels were isolated as described in Pardridge, et al., "Rapid Sequestration And Degradation Of Somatostatin Analogues By Isolated Brain Microvessels", (Journal of Neurochemistry, Vol. 44, No. 4, 1985, pp. 1178-1184).

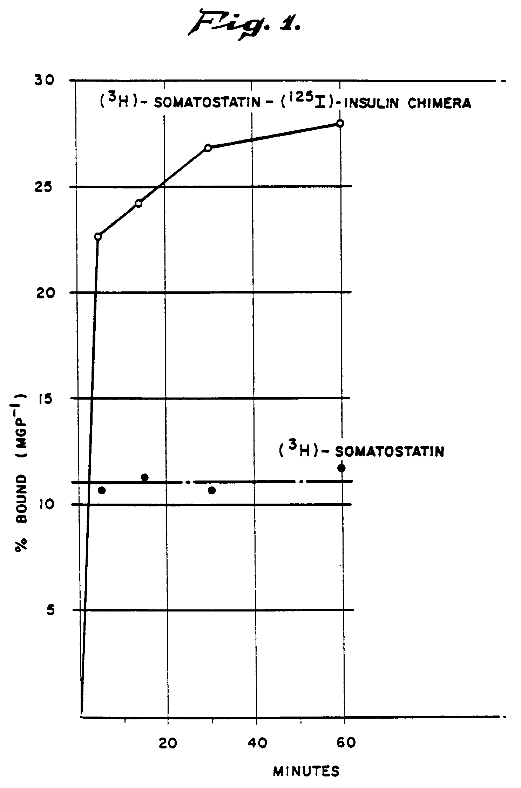

³H-somatostatin was added to one set of microvessels for up to 60 minutes incubation at room temperature. In another set of incubations, the ³H-somatostatin-¹²⁵I-insulin chimera was also added. As shown in Fig. 1, the uptake of the chimera was more than double that of the free somatostatin. Moreover, the uptake of the chimera increased with time, whereas there was no increase in time with the free somatostatin. The uptake of the free somatostatin likely represents nonspecific binding as described in the article mentioned above (Journal of Neurochemistry, Vol. 44, No. 4, 1985).

This example demonstrates the receptor-mediated transcytosis or endocytosis of somatostatin-insulin chimera via the insulin receptor. Previous studies have shown that the receptor-mediated endocytosis of peptides in the isolated brain microvessels is a reliable index of the in vivo blood-brain barrier receptor transport activity of peptides in vivo (see my previously-mentioned article in Endocrine Reviews, Vol. 7, No. 3, August 1986).

### Example 3:

A chimeric peptide is prepared according to the same procedure as in Example 1 except that transferrin is substituted for insulin. The resulting chimeric peptide is combined with sterile saline to provide a solution containing 0.01 weight percent chimeric peptide which is administered to the patient parenterally or intranasally.

### Example 4:

A chimeric peptide is prepared according to the same procedure as in Example 1 except that vasopressin is substituted for somatostatin. The resulting chimeric peptide is combined with sterile saline to provide a solution containing 0.01 weight percent chimeric peptide which is administered to the patient parenterally.

### Example 5:

A chimeric peptide is prepared according to the same procedure as in Example 1 except that transferrin is coupled to alpha-interferon. The resulting chimeric peptide is combined with sterile saline to provide a solution containing 0.01 weight percent chimeric peptide which is administered to the patient or subject parenterally or intranasally.

### Example 6:

A chimeric peptide is prepared according to the same procedure as in Example 1 except that IGF-II is coupled to beta-endorphin. The resulting chimeric peptide is combined with sterile saline to provide a solution containing 0.01 weight percent chimeric peptide which is administered to the patient or subject parenterally or intranasally.

### Example 7:

A chimeric peptide is prepared according to the same procedure as in Example 1 except that insulin is coupled to ACTH 4-9 analogue. The resulting chimeric peptide is combined with sterile saline to provide a solution containing 0.01 weight percent chimeric peptide which is administered to the patient or subject parenterally or intranasally.

### Example 8:

A chimeric peptide is prepared according to the same procedure as in Example 1 except that cationized albumin is coupled to hexosaminidase A. The resulting chimeric peptide is combined with sterile saline to provide a solution containing 0.01 weight percent chimeric peptide which is administered to the patient or subject parenterally or intranasally.

### Example 9:

A chimeric peptide is prepared according to the same procedure as in Example 1 except that commercially available bovine histone type V is substituted for insulin. The resulting chimeric peptide is combined with sterile saline to provide a solution containing 0.01 weight percent chimeric peptide which is administered to the patient parenterally or intranasally.

### Example 10:

To demonstrate the usefulness of histone as a polycationic transportable peptide, the following uptake tests were conducted.

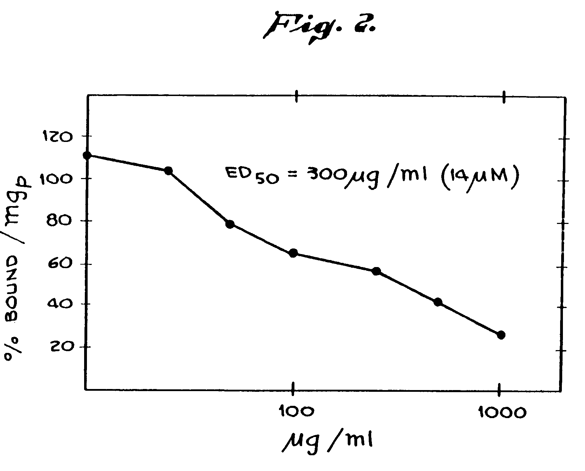

A radio receptor assay with bovine brain capillaries was conducted according to the procedure set forth in Example 2, except that commercially supplied bovine histone type V, instead of the somatostatin-insulin chimera, was radiolabeled with ¹²⁵I-iodine and chloramine-T. The histone was incubated with the brain capillaries for 60 minutes and the percent uptake per mg brain capillaries was determined. The results of these tests are depicted in Fig. 2 and they show that the uptake of the histone was high, approximating 110% bound per mg protein (background binding is 3-5% per mg protein). Also, it was found and Fig. 2 shows that the uptake of the histone is readily saturable by increasing concentrations of unlabeled histone and that the 50% inhibition point is reached at a concentration of 300 ug/ml or approximately 14 uM. This saturation response is typical of a receptor-mediated or absorptive-mediated endocytosis mechanism.

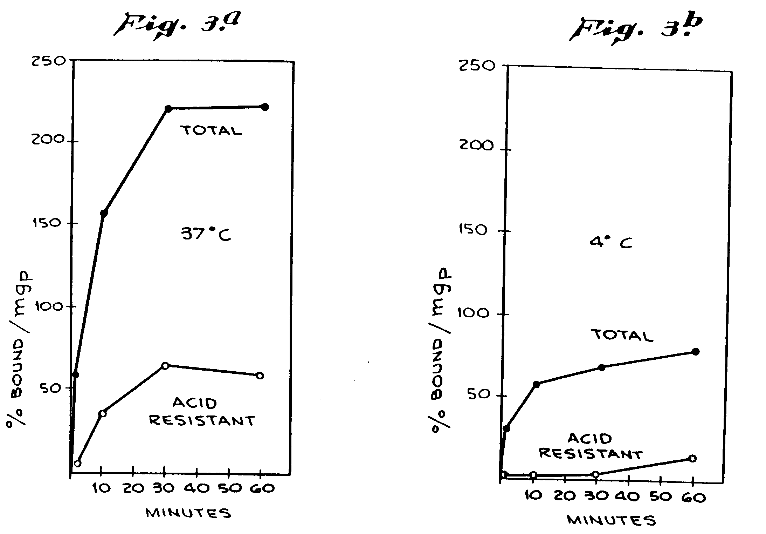

The amount of histone V uptake was measured at 2, 10, 30 and 60 minute intervals. It was found that the uptake of the histone increases with time as shown in both Fig. 3a and Fig. 3b which relate to incubations of histone V conducted at 37°C. and 4°C. respectively. Histone uptake was however inhibited at 4°C. Further, histone uptake resistance to acid wash was measured by treating the bovine brain capillaries with a mild acid wash after histone uptake was completed. The acid wash was accomplished by rinsing the brain capillaries in a solution of cold 0.028 molar sodium acetate, 0.02 molar sodium barbital and 0.12 molar sodium chloride having a pH of about 3.0. The results of these tests showed that the uptake of histone by the isolated brain capillaries is partially resistant to the mild acid wash. See the graphic results in Figs. 3a and 3b, showing acid resistant histone uptake. The results indicate that about one-third of the histone taken up is actually endocytosed into the brain capillaries. The above results showing that both binding and endocytosis are slowed by incubation at 4°C. is typical of a receptor mediated-mediated or adsorptive-mediated uptake mechanism for transport across the blood-brain barrier.

A one nanomolar concentration of the ¹²⁵I-histone V was incubated at room temperature for 10 minutes in the presence of brain capillaries. The amount of labeled histone taken up by the brain capillaries was then determined. The results of the tests showed that the uptake of the ¹²⁵I-histone V is linear with respect to the amount of capillary protein in the incubation flask. The results of these tests are shown graphically in Fig. 4.

### Example 11 -

This example demonstrates the preparation of a histone/horseradish peroxidase conjugate which was tested and shown to be sequestered by brain microvasculature. The histone/horseradish peroxidase conjugate was prepared as follows.

Sulfhydryl groups were introduced onto histone by a modification of the procedures described by Jue et al. (1978) Biochemistry 17:5399-5405. Sixteen milligrams of histone (Sigma type II-AS) was dissolved in 3.0 ml of 50 mM triethanolamine buffer, pH 8.25, in a sealed reaction vessel. The protein solution was exhaustively degassed and purged with nitrogen. To this was added 0.074 ml of a 0.5 M solution of 2-iminothiolane (Pierce) via syringe. The mixture was allowed to react for 45 minutes at 25°C. In order to reduce all sulfhydryls, the product was then incubated with 0.5 ml of a 0.1 M dithiothreitol (Calbiochem) for 30 min. at 37°C with mild shaking (75 rpm). The modified protein was purified from the reaction mixture by chromatography over Sephadex G-25 (1.5 x 30 cm) using degassed, nitrogen purged triethanolamine buffer. Protein elution was monitored by UV absorbance at 280 nm [5-5'Dithio-bis-(2-nitrobenzoic acid), Ellman (1959) Arch. Biochem.Biophys. 82:70-77]. Typically, 1.1 moles of sulfhydryl-/mole of protein were introduced using these conditions.

Horseradish peroxidase (HRP) was derivatized with maleimide groups using N-γ-maleimidobutyryloxysuccinimide (GMBS) by a method described by Hashida et al. (1984) J. Appl. Biochem. 6:56-63. To 20 mg of peroxidase (0.5 mmoles, Boeringer Mannheim EIA grade) was added 1.5 ml of 0.1 M sodium phosphate buffer, pH 7.0. Sixteen mg (57 mmoles) GMBS was dissolved in 0.2 ml of DMF and added to the peroxidase solution. The mixture was shaken for 30 minutes at 30°C. Derivatized HRP was isolated by chromatography of the reaction mixture over Sephadex G-25 (1.5 x 30 cm) using 0.1 M phosphate, pH 6 as an eluant. HRP elution was monitored by its absorbance at 403 nM.

The pH of the thiolated histone solution was adjusted to 6 with 1 N HCl. This was immediately added to the maleimide derivatized HRP. The solution was exhaustively degassed, nitrogen purged and septum sealed. The components were allowed to react for 48 hours at 25°C. Separation of the conjugate from unreacted proteins was achieved on a Sephadex G 100 SF column (2.5 x 54 cm) using 0.1 M phosphate, pH 6 as an eluant. Fractions isolated from the column were checked for absorbance at 230 nm and 403 nm. The first absorbing eluant was pooled, dialyzed against water at 4°C overnight and lyophilized. Following reconstitution in 2 mL of PBS, 80% (16 mg) of the peroxidase was isolated with the conjugate peak. SDS-page analysis on the conjugate revealed several bands of MW 54 kD and above and could not detect any free HRP or histone. Furthermore, total protein, as determined by the method of Lowry, indicated 1/3 of the mass could be accounted for by HRP suggesting at 6/1 histone/HRP conjugation ratio. The conjugate was adjusted to 1 mg/ml (total protein by Lowry method) and stored at 4°C prior to in vivo analysis.

The histone/HRP conjugate was tested as follows:
BALB/C mice (6 weeks, female) were injected intravenously with 0.2 ml of histone/HRP conjugate in a phosphate buffered saline (PBS). Animals were killed after 15 minutes with a lethal injection of chlorohydrate, perfused with 5.0 ml of PBS containing 4% paraformaldehyde, and then the brain was removed and placed into fixative for an additional 15 minutes. Frozen sections (30 microns thick) were prepared and floated in PBS for at least 20 minutes. The sections were removed from PBS and placed in incubation solution [20 mm sodium acetate, pH 3.3, 2.5% ethanol, 4mM sodium nitroprusside, 250 mM 3,3',5,5'- tetramethyl benzidine (TMB)] for 20 minutes. The reaction was initiated by the addition of 1 ml of 0.3% H₂O₂ to each 100 ml of incubation solution. The reaction was allowed to continue for 10 minutes at room temperature and then the sections were transferred to 20 mM sodium acetate pH 3.3. The sections were washed six times for five minutes each. Tissue sections were mounted onto gelatin coated slides and allowed to dry for seven hours at room temperature. The slides were heated at 60°C for 1 hour, and stained for 30 seconds with 0.5% toluidine blue pH 4.5. The slides were dehydrated in a graded alcohol series, washed with xylene, and mounted with Permount.

The capillaries of the brain were visualized by the presence of the TMB reaction product associated with the luminal surface of the capillary. The product was observed in animals injected with the histone/HRP conjugate, but was not observed in animals injected with either HRP alone or in PBS injected animals. Both white and gray matter were labeled with TMB. There was no overt appearance of specific localization to only highly vascularized areas of the brain.

### Example 12 -

This example sets forth experiments which demonstrate that histone is capable of penetrating the blood-brain barrier (BBB) in vivo. Calfthymus histone was iodinated with [¹²⁵I]-iodine and was found to be rapidly taken up by isolated bovine brain capillaries used as an in vitro model system of the BBB via a time- and temperature-dependent mechanism. The binding was saturable and a Scatchard plot of the binding data was linear, yielding a ${\text{K}}_{\text{D}} \text{= 15.2 ± 2.8 µM}$ and a maximal binding (Bmax) = 7.7 ± 1.0 nmol/mg protein (Fig. 5).

Other polycations such as protamine or polylysine markedly inhibited uptake of [¹²⁵I]-histone, but cationized albumin demonstrated minimal inhibition and cationized immunoglobulin caused no inhibition of bovine brain capillary uptake of [¹²⁵I]-histone. The in vivo brain volume of distribution of [¹²⁵I]-histone reached 159 ± 70 µL/g by ten minutes of carotid arterial perfusion as compared to the 10 minute volume of distribution for [³H]-albumin, 17 ± 7 µL/g. Most of this uptake represented sequestration by the vasculature, but approximately 8% of the total histone taken up by brain was found to be transported unmetabolized (based on trichloroacetic acid (TCA) precipitability) into brain interstitium.

The experiments were conducted as follows:

### METHODS

### Materials

[¹²⁵I]-iodine was obtained from DuPont-New England Nuclear Corporation (Boston, MA). [³H]-NaBH₄ was purchased from Amersham Corporation (Chicago, IL). Bovine albumin (Pentex fraction V) was obtained from Miles Laboratories (Elkhart, IN). Male, Sprague-Dawley rats (200-300 g) were obtained from Bantin and Kingman (Fremont, CA). Calf thymus histone VS (lysine-rich) and all other reagents were obtained from Sigma Chemical Company (St. Louis, MO).

### Histone Iodination

Histone was iodinated to a specific activity of 10-20 µCi/µg using [¹²⁵I]-iodine and chloramine T. Fifty µg of histone (2.3 nmol) were reacted with 2.5 mCi of [¹²⁵I]-iodine (1.2 nmol) and 2.1 nmol chloramine T followed by a 60 second incubation at room temperature. The mixture was acidified with 0.01 N HCl and applied to an 0.7 x 28 cm column of Sephadex G-25 (medium) and 0.01 N HCl. The iodinated histone eluted in the void volume and was 98% precipitable with trichloroacetic acid (TCA). The [¹²⁵I]-histone was stored at 4°C in 0.01 N HCl, but was subject to relatively rapid de-iodination over the course of a week. Therefore, the in vivo carotid artery perfusion experiments were performed with 24 hours of iodination, and the isolated capillary experiments were performed with 3 to 4 days of iodination.

### Tritiation of Albumin

Bovine albumin (Pentex fraction V) was tritiated to a specific activity of 0.4 µCi/µg with [³H]-NaBH₄ as described previously (Pardridge et al., 1985a). The TCA precipitability of this preparation was >99%.

### Brain Microvessel Experiments

Bovine brain microvessels were isolated with a mechanical homogenization technique, as described previously (Pardridge et al.: Rapid sequestration and degradation of somatostatin analogues by isolated brain microvessels. J. Neurochem. **44**: 1178-1184, 1985) from fresh bovine cortex obtained from a local slaughterhouse. The final microvessel pellet was cryopreserved in 0.28 M sucrose, 0.02 M Tris (pH 7.4) and 2 mM dithiothreitol in liquid nitrogen (-70°C). On the day of the experiment, the microvessels were thawed, centrifuged, and resuspended in Ringer-HEPES buffer (RHB) (10 mM HEPES, pH 7.4, 141 mM NaCl, 4 mM KCl, 2.8 mM CaCl₂ and 0.1 gm/dl bovine serum albumin). Uptake of [¹²⁵I]-histone by bovine brain microvessels was performed as described previously (Pardridge et al.: Cationization of immunoglobulin G (IgG) as a new strategy for enhanced IgG delivery through the blood-brain barrier. Clin. Res. 37: 140A). Briefly, approximately 100 µg of capillary protein was incubated with 0.2 µCi/ml of [¹²⁵I]-histone in a final volume of 0.45 ml RHB at 37°C or 4°C for time periods ranging from 5 seconds to 60 minutes. Competitive binding studies were performed by adding various concentrations of either unlabeled histone, cationized immunoglobulin, native or cationized bovine serum albumin, protamine, or polylysine (59,000 molecular weight). At the end of the incubation period, the mixture was centrifuged at 10,000 g for 45 seconds and the capillary pellet was solubilized in 0.5 ml of 1 N NaOH, followed by [¹²⁵I] counting and protein determination by the method of Lowry et al. (Protein measurement with the Folin phenol reagent. J. Biol. Chem. 193: 262-275, 1951).

Internalization of the labeled histone by the isolated bovine brain capillaries was assessed by a mild acid wash assay as described previously (Pardridge et al., Rapid sequestration and degradation of somatostatin analogues by isolated brain microvessels. J. Neurochem. 44: 1178-1184, 1985). The acid wash solution consisted of 0.12 M NaCl, 0.02 M sodium acetate (pH = 3) and 0.028 M of sodium barbital.

### [¹²⁵I]-Histone Transport Through the BBB In Vivo

Quantitation of in vivo transport of [¹²⁵I]-histone through the BBB in vivo was determined with an internal carotid artery perfusion technique coupled with a capillary depletion method. Rats were anesthetized with ketamine/xylazine (ketamine, 200 mg/kb, i.p./xylazine, 2 mg/kg, i.p.) and, following exposure of the right common carotid artery, the occipital, superior thyroid, and pterygopalatine arteries were closed by electrocoagulation. The right external carotid artery was catheterized with a polyethylene catheter (PE-10). The common carotid artery was tied just before the perfusion was started and was kept closed. The perfusion consisted of Krebs-Henseleit buffer, pH 7.4 (118 mM NaCl, 4.7 mM KCl, 2.5 mM CaCl₂, 1.2 mM MgSO₄, 1.2 mM KH₂PO₄, 25 mM NaHCO₃, 10 mM D-glucose and 3 gm/dl bovine serum albumin), containing 2.5 µCi/ml of [¹²⁵I]-histone and 25 µCi/ml of [³H]-albumin. The perfusate was maintained at 37°C and was continuously oxygenated during the perfusion, which was carried out at a 1-1.2 ml/min flow rate (Harvard peristaltic pump Model 1210) for 1 to 10 minutes. For perfusion times longer than 2.5 minutes, the rat blood volume was maintained constant by withdrawing blood from the femoral artery (through a PE-50 catheter filled with heparin) at the same flow rate (Harvard syringe pump Model 940). Following the perfusion, the animals were decapitated and the ipsilateral brain hemisphere was removed. The choroid plexus was discarded, and the brain was weighed and then homogenized in 3.5 ml of physiologic buffer, pH = 7.4 (10 mM HEPES, 141 mM NaCl, 4 mM KCl, 2.8 mM CaCl₂, 1 mM MgSO₄, 1 mM NaH₂PO₄, 10 mM D-glucose). Four ml of 26% dextran solution (79,000 molecular weight) was added to a final dextran concentration of 13%, and the material was re-homogenized (3 strokes). All of the homogenization procedures were performed at 4°C.

After removing an aliquot of the homogenate for radioisotope counting, the remainder was centrifuged at 5,400 g for 15 minutes at 4°C in a swinging bucket rotor (Beckman JA-7.5 rotor, Beckman J2-21 centrifuge). The supernatant and pellet were carefully separated. Microscopic examination of the pellet showed that it consisted of brain vasculature, red cells, and brain nuclei, whereas the supernatant was essentially devoid of vasculature. Aliquots of the supernatant fraction and perfusate (to which was added an amount of dextran similar to that present in the supernatant) were taken for 25% TCA precipitability. Homogenate, pellet, supernatant, and perfusate samples were solubilized in 2 ml Soluene-350 (Packard Instrument Co., Downers Grove, IL) and prepared for [¹²⁵I], [³H] double isotope liquid scintillation spectrometry as described previously (Pardridge, Carrier-mediated transport of thyroid hormones through the blood-brain barrier. Primary role of albumin-bound hormone. Endocrinology 105: 605-612, 1979.)

Volumes of distribution for both the [¹²⁵1]-and the [³H]-labeled proteins were calculated for the homogenate, pellet, and the postvascular supernatant: 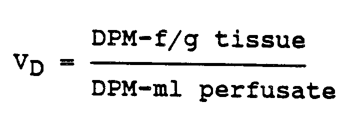
where DPM-f = the DPM in the respective fraction (i.e., homogenate, pellet, or postvascular supernatant).

The supernatant volumes of distribution were corrected as follows:

${\text{Supernatant V}}_{\text{D}} {\text{= {[¹²⁵I] V}}_{\text{D}} {\text{x % TCA precipitability}- {[³H] V}}_{\text{D}} \text{}}$

The corrected supernatant V_{D} is a quantitative measure of protein distribution into the brain interstitium following transcytosis across the BBB. The subtraction of the V_{D} for [³H]-albumin was found to be necessary to correct for radioactive protein contained within the lumen of the brain vasculature that leaks from the vessels following homogenization and rupture of these vessels.

### Clearance of [¹²⁵I]-Histone and [³H]-Albumin Following a Single Intravenous Injection

An 0.5 ml aliquot of physiologic buffer containing 5 µCi of [¹²⁵I]-histone and 50 µCi of [³H]-native albumin was rapidly injected into a femoral vein through a 27-gauge needle. At 0.25, 5, 30, 60, 120 and 180 minutes after the injection, the animal was quickly laparotomized and an 0.5 ml aliquot of arterial blood was removed from the descending aorta followed by decapitation of the animal and extirpation of the brain and nine other organs (heart, liver, spleen, testis, small intestine, skeletal muscle, fat, kidney and lung). The tissues were solubilized in Soluene-350 and analyzed with double isotope liquid scintillation counting. The blood [³H] and [¹²⁵I] radioactivities were normalized to DPM/ml as a percent of injected dose, i.e., A(t), and these data were fit to the following biexponential function: 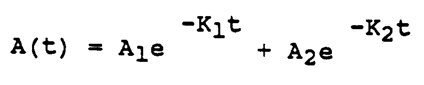
using a derivative-free nonlinear regression analysis (PAR of BMDP, Biomedical Computer P Series Programs developed at UCLA Health Sciences Computing Facility). Because the standard error was roughly proportional to the means, the data were weighted using weight = 1/[clearance]. The integral of the arterial radioactivity curve was determined from these data as follows: 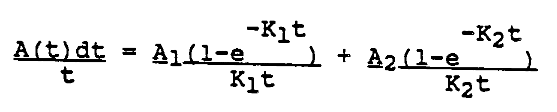
where t = time after injection. The volume of distribution of histone or albumin in brain and the nine other organs was determined from the ratio of DPM/gm tissue divided by integrated DPM/ml blood. The TCA precipitability of the serum [³H]-albumin was greater than 98% at all time points. However, there was a progressive decrease in the TCA precipitability of the [¹²⁵I]-histone. Therefore, only arterial TCA precipitable [¹²⁵I] counts were used in computation of the clearance of histone from blood or the organ volumes of distribution.

### Uptake by Bovine Brain Microvessels

[¹²⁵I]-histone was rapidly taken up by isolated bovine brain capillaries at 37°C, and approximately 25% of this uptake was resistant to mild acid wash and is presumed to represent internalized histone (see Fig. 3). Both the total binding and internalization were slowed by incubation at 4°C (see Fig. 3). The uptake of the [¹²⁵I]-histone by isolated bovine brain capillaries was linear with respect to the amount of capillaries added to the incubation vessel throughout the range of 47-210 µg of capillary protein (data not shown). The binding of the [¹²⁵I]-histone to the isolated brain microvessels was saturable with an ED₅₀ of approximately 300 µG/ML (14 µM) as shown by the data in Fig. 2. These saturation data were analyzed by Scatchard analysis to give the plot in Fig. 5. The dissociation constant

(K_{D}) = 15.2 ± 2.8 µM

and the maximal binding or Bmax = 7.7 ± 1.0 nmol/mg protein. The binding of [¹²⁵I]-histone to isolated bovine brain microvessels was inhibited by other polycationic proteins such as protamine or polylysine, but was minimally inhibited by cationized albumin and was not inhibited by cationized immunoglobulin G or native albumin (see Table 1).

**Table 1**

| Competition for [¹²⁵I]-Histone Binding to Isolated Bovine BRain Capillaries In Vitro | |
|---|---|
| Medium | % Bound/mgₚ of [¹²⁵I]-Histone |
| Control | 225 ± 10 |
| 2.5 mg/ml cationized immunoglobulin | 232 ± 2 |
| 2.5 mg/ml native albumin | 211 ± 9 |
| 0.5 mg/ml cationized albumin | 171 ± 13^{a} |
| 0.5 mg/ml histone | 113 ± 4^{b} |
| 0.5 mg/ml protamine | 95 ± 5^{b} |
| 2.5 mg/ml polylysine (59,000) | 46 ± 1^{b} |
| Data are mean ± S.E. (n = 3). | |

| | |
|---|---|
| aₚ < 0.01. | |
| bₚ < 0.0005. | |

### Transport Through The Rat BBB In Vivo

The homogenate V_{D} for [¹²⁵I]-histone increased with time and reached 159 ± 70 µLg⁻¹ by 10 minutes (see Table 2). Most of this [¹²⁵I]-histone taken up by brain, however, was sequestered in the vascular compartment, as the postvascular supernatant V_{D} of [¹²⁵I]-histone at 10 minutes was 12 ± 5 µLg⁻¹, which represents 8% of the total uptake. The [¹²⁵I]-histone in the supernatant that was TCA precipitable was 90 ± 3% by 10 minutes of perfusion. The homogenate V_{D} for [¹²⁵I]-histone was nearly ten-fold greater than the homogenate V_{D} for [³H]-albumin (see Table 2). By definition, the supernatant V_{D} for [³H]-albumin = 0. The pellet V_{D} for [³H]-albumin was 0.76 ± 0.20 µLg⁻¹ at 10 minutes of perfusion, which is >100-fold less than the pellet V_{D} for [¹²⁵I]-histone (see Table 2).

**Table 2**

| Volume of Distribution (V_{D}) of [¹²⁵I]-Histone or [³H]-Albumin after 1 to 10 Minute Perfusions in Rat Brain In Vivo | | | | | |
|---|---|---|---|---|---|
| Protein | Brain Fraction | Time (min) | | | |
| | | 1 | 2.5 | 5 | 10 |
| [¹²⁵I]-histone | Homogenate | 5.1±3.5 | 20±10 | 64±13 | 159±70 |
| | Supernatant | 1.6±0.5 | 1.4±0.1 | 12±4 | 12±5 |
| | Pellet | 0.9±0.5 | 10±8 | 39±9 | 118±59 |
| [³H]-albumin | Homogenate | 2.2±0.7 | 2.8±0.4 | 18±7 | 17±7 |
| Data are mean ± S.E. (n = 3-7). Reported as V_{D}, µL/g. | | | | | |

### Clearance of [¹²⁵I]-Histone and [³H]-Albumin from Blood following a Single Intravenous Injection

The decay is plasma [¹²⁵I]-histone that was TCA precipitable and the decay in plasma [³H]-albumin following a single intravenous injection is shown in Fig. 6. The [³H]-albumin data could not be fit to a biexponential function, but did fit a monoexponential function. The [¹²⁵I]-histone blood data could not be fit to either a monoexponential or a triexponential function but did fit to a bioexponential function, and the intercepts and slopes of the two exponential decays are given in Fig. 6.

Following rapid clearance from blood, [¹²⁵I]-histone was cleared monoexponentially with a half-time of 2.0 ± 0.5 hours, and this value was about 40% of the half-time for [³H]-albumin clearance, 4.8 ± 1.8 hours (see Fig. 6). The 60-minute brain V_{D} of [¹²⁵I]-histone and the ratios of the 60-minute [¹²⁵I]-histone V_{D}/[³H]-albumin V_{D} for brain and nine other organs are shown in Table 3. The 60-minute V_{D} data are shown because, with the exception of testis and small intestine, brain and the other organs reached maximal organ distribution by 60 minutes. The 180-minute V_{D} was 41% and 53% higher than the 60-minute V_{D} for testis and small intestines, respectively. The 180-minute V_{D} was 40% lower than the 60-minute V_{D} for brain, lung, and spleen. The V_{D} was essentially unchanged at 60 or 180 minutes for liver, heart, kidney, muscle, or fat.

**Table 3**

| Volumes of Distribution (V_{D}) of Histone and Albumin at 60 Minutes Following Intravenous Injection | | | |
|---|---|---|---|
| Organ | Histone V_{D} (ml/g) | Albumin V_{D} (ml/g) | Ratio of V_{D}-Histone/V_{D}-Albumin |
| Kidney | 7.9±0.5 | 0.36±0.02 | 22.3±1.4 |
| Muscle | 0.71±0.01 | 0.036±0.002 | 19.6±0.2 |
| Spleen | 6.0±0.8 | 0.35±0.03 | 17.5±2.4 |
| Small | | | |
| Intestine | 2.0±0.7 | 0.11±0.01 | 17.4±6.1 |
| Liver | 4.6±0.2 | 0.34±0.02 | 13.7±0.7 |
| Lung | 5.1±0.3 | 0.38±0.03 | 13.3±0.7 |
| Brain | 0.17±0.02 | 0.017±0.002 | 10.0±1.2 |
| Testis | 0.68±0.04 | 0.086±0.011 | 7.9±0.5 |
| Fat | 0.32±0.02 | 0.048±0.006 | 6.7±0.5 |
| Heart | 0.94±0.10 | 0.21±0.03 | 4.5±0.5 |
| V_{D} shown are data obtained 60 minutes after single intravenous injection. Data are mean ± S.E. (n = 3). | | | |

This example demonstrates that [¹²⁵I]-histone binds both the lumenal and antilumenal sides of the brain capillary. The in vivo perfusion studies in Table 2 showing the very high microvascular pellet V_{D} for [¹²⁵I]-histone relative to [³H]-albumin, demonstrate that [¹²⁵I]-histone is bound by the lumenal membrane of the brain capillary. Conversely, there must also be binding to the antilumenal membrane to explain the rapid binding within 5 seconds of incubation with isolated bovine brain microvessels (see Fig. 3).

The binding of the [¹²⁵I]-histone to brain microvessels is temperature-dependent (Fig. 3) and is saturable (Fig. 2). The saturation ED₅₀ of 14 µM histone indicates that the capacity of the histone uptake system is very high. For example, the saturation ED₅₀ for cationized albumin was 0.05 mg/ml (0.7 µM) (Kumagai et al.: Absorptive-mediated endocytosis of cationized albumin and a β-endorphin-cationized albumin chimeric peptide by isolated brain capillaries. Model system of blood-brain barrier transport. J. Biol. Chem. 262: 15214-15219, 1987), and for cationized immunoglobulin was 1 mg/ml (6 µM) (Triguero et al.: Cationization of immunoglobulin G (IgG) as a new strategy for enhanced IgG delivery through the blood-brain barrier. Clin.Res. 37: 140A). The Bmax for histone of 7.7 ± 1.0 nmol/mg protein is approximately five-fold the Bmax for cationized immunoglobulin G and is approximately ninety-fold greater than the Bmax for binding of cationized albumin. The differing Bmax values for the various polycationic proteins suggests that these molecules bind to different groups of negative charges on the brain capillary endothelial membrane. For example, cationized albumin only weakly inhibits histone uptake, whereas cationized immunoglobulin has no effect, as polylysine (see Table 1).

Histone undergoes absorptive-mediated endocytosis into brain capillary endothelial cytoplasm following its binding to the surface of the capillary, as demonstrated by the resistance to mild acid wash (Fig. 3). Moreover, the data in Table 2 show that approximately 8% of the total histone bound and endocytosed by the brain capillary undergoes exocytosis into the brain interstitium in vivo, which completes an overall pathway of transcytosis through the capillary endothelium.

## Claims

1. A chimeric peptide adapted for delivering a neuropharmaceutical agent into the brain by transcytosis through the blood-brain barrier, said chimeric peptide comprising a transportable peptide capable of crossing the blood-brain barrier by transcytosis conjugated with said neuropharmaceutical agent, wherein said transportable peptide is histone.

2. A chimeric peptide according to claim 1 wherein said histone is isolated from a human source.

3. A chimeric peptide according to claim 1 or 2, wherein said histone is selected from a class I-V type of histone.

4. A chimeric peptide according to any preceding claim wherein said neuropharmaceutical agent is a hydrophilic peptide.

5. A chimeric peptide according to claim 4 wherein said neuropharmaceutical agent is selected from the group consisting of somatostatin, thyrotropin releasing hormone, vasopressin, alpha interferon, endorphin, muramyl dipeptide and L-methionyl(sulfone)-L-glytamyl-L-histidyl-L-phenylalanyl-D-lysyl-L-phenylalanine.

6. A chimeric peptide according to any preceding clam wherein said transportable peptide and neuropharmaceutical agent are conjugated via a conjugation agent.

7. A chimeric peptide according to claim 6 wherein said conjugation agent is capable of conjugating the transportable peptide to said neuropharmaceutical agent by peptide thiolation or lysine coupling via glutaraldehyde.

8. A chimeric peptide according to claim 1 having the formula 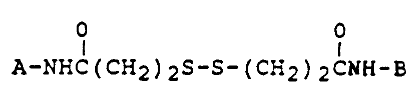 wherein A is a neuropharmaceutical agent and B is histone.

9. A chimeric peptide according to claim 8 wherein A is selected from the group consisting of somatostatin, thyrotropin releasing hormone, vasopressin, alpha interferon, endorphin, muramyl dipeptide and L-methionyl(sulfone)-L-glytamyl-L-histidyl-L-phenylalanyl-D-lysyl-L-phenylalanine.

10. A composition comprising a chimeric peptide according to any preceding clam and a pharmaceutically acceptable carrier for said chimeric peptide.

11. A composition according to claim 10 wherein said pharmaceutically acceptable carrier is sterile saline.

## Patentansprüche

1. Schimärisches Peptid zum Befördern eines neuropharmazeutischen Agens in das Hirn durch Transcytose durch die Blut-Hirn-Barriere, enthaltend ein transportables zum Durchqueren der Blut-Hirn-Barriere durch Transcytose befähigtes Peptid, das mit dem neuropharmazeutischen Agens konjugiert ist, wobei als transportables Peptid ein Histon vorgesehen ist.

2. Schimärisches Peptid nach Anspruch 1,
bei dem das Histon aus einer Humanquelle isoliert ist.

3. Schimärisches Peptid nach Anspruch 1 oder 2,
bei dem das Histon ausgewählt ist aus den Histonen der Klassentypen I - V.

4. Schimärisches Peptid nach einem der vorangehenden Ansprüche, bei dem das neuropharmazeutische Agens ein hydrophiles Peptid ist.

5. Schimärisches Peptid nach Anspruch 4,
bei dem das neuropharmazeutische Agens ausgewählt ist aus der Gruppe, bestehend aus Somatostatin, Thyrotropin freisetzendes Hormon (TRH), Vasopressin, Alpha-Interferon, Endorphin, Muramyl-Dipeptid und L-methionyl(sulfon)-L-glutamyl-L-histidyl-L-phenylalanyl-D-lysyl-L-phenylalanin.

6. Schimärisches Peptid nach einem der vorangehenden Ansprüche, bei dem das transportable Peptid und das neuropharmazeutische Agens über ein Kopplungsagens konjugiert sind.

7. Schimärisches Peptid nach Anspruch 6,
bei dem das Kopplungsagens fähig ist, das transportable Peptid mit dem neuropharmazeutischen Agens über Peptid-Thiolierung oder Lysin-Kopplung über Glutaraldehyd zu konjugieren.

8. Schimärisches Peptid nach Anspruch 1 einer Formel 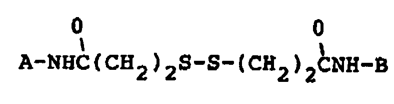 worin A ein neuropharmazeutisches Agens und B ein Histon ist.

9. Schimärisches Peptid nach Anspruch 8,
bei dem A ausgewählt ist aus der Gruppe, bestehend aus Somatostatin, Thyrotropin freisetzendes Hormon (TRH), Vasopressin, Alpha-Interferon,
Endorphin, Muramyl-Dipeptid und
L-methionyl(sulfon)-L-glutamyl-L-histidyl-L-phenylalanyl-D-lysyl-L-phenylalanin.

10. Komposition, enthaltend ein schimärisches Peptid gemäß einem der vorangehenden Ansprüche und einen pharmazeutisch akzeptablen Träger für das schimärische Peptid.

11. Komposition nach Anspruch 10,
bei der der pharmazeutisch akzeptable Träger eine sterile Salzlösung ist.

## Revendications

1. Peptide chimérique adapté pour libérer un agent neuropharmaceutique dans le cerveau, par transcytose à travers la barrière hémato-encéphalique, ledit peptide chimérique comportant un peptide transportable, capable de traverser la barrière hémato-encéphalique par transcytose, conjugué avec ledit agent neuropharmaceutique, et ledit peptide transportable étant une histone.

2. Peptide chimérique conforme à la revendication 1, dans lequel ladite histone a été isolée à partir d'une source humaine

3. Peptide chimérique conforme à la revendication 1 ou 2, dans lequel ladite histone est choisie dans la classe des types I-V d'histones.

4. Peptide chimérique conforme à l'une des revendications précédentes, dans lequel ledit agent neuropharmaceutique est un peptide hydrophile.

5. Peptide chimérique conforme à la revendication 4, dans lequel ledit agent neuropharmaceutique est choisi dans le groupe constitué par la somatostatine, une hormone libérant de la thyrotropine, la vasopressine, l'interféron alpha, une endorphine, un muramyl-dipeptide et la L-méthionyl(sulfone)-L-glutamyl-L-histidyl-L-phénylanalyl-D-lysyl-L-phénylalanine.

6. Peptide chimérique conforme à l'une des revendications précédentes, dans lequel ledit peptide transportable et ledit agent neuropharmaceutique sont conjugués par l'intermédiaire d'un agent de conjugaison.

7. Peptide chimérique conforme à la revendication 6, dans lequel ledit agent de conjugaison est capable de conjuguer le peptide transportable à l'agent neuropharmaceutique par thiolation de peptide ou couplage de lysine par l'intermédiaire de glutaraldéhyde.

8. Peptide chimérique conforme à la revendication 1, de formule 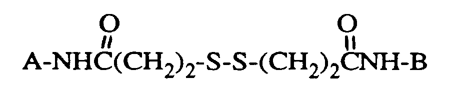 dans laquelle A représente un agent neuropharmaceutique et B représente une histone.

9. Peptide chimérique conforme à la revendication 8, dans lequel A est choisi dans le groupe constitué par la somatostatine, une hormone libérant de la thyrotropine, la vasopressine, l'interféron alpha, une endorphine, un muramyl-dipeptide et la L-méthionyl(sulfone)-L-glutamyl-L-histidyl-L-phénylanalyl-D-lysyl-L-phénylalanine.

10. Composition comprenant un peptide chimérique conforme à l'une des revendications précédentes et un véhicule pharmaceutiquement acceptable pour ledit peptide chimérique.

11. Composition conforme à la revendication 10, dans laquelle ledit véhicule pharmaceutiquement acceptable est une solution salée stérile.
